# FASCICULE DE BREVET EUROPEEN

(11) **EP 3 280 338 B1**
(45) Date de publication et mention de la délivrance du brevet: **10.06.2020**
(21) Numéro de dépôt: 16718216.1
(22) Date de dépôt: 05.04.2016
(51) Int. Cl.: A61B 17/34, A61F 2/24, A61B 17/3207, A61N 1/05

(54) **ENSEMBLE DE REMPLACEMENT D'UNE VALVE CARDIAQUE COMPRENANT UN CATHETER DE DELIVRANCE AVEC OU SANS INTRODUCTEUR**
ANORDNUNG ZUM ERSETZEN EINER HERZKLAPPE MIT EINEM EINFÜHRKATHETER MIT ODER OHNE EINFÜHRHILFE
ASSEMBLY FOR REPLACING A HEART VALVE INCLUDING A DELIVERY CATHETER WITH OR WITHOUT INTRODUCER

(30) Priorité: 07.04.2015 FR 1552955; 10.07.2015 FR 1556582
(43) Date de publication de la demande: 14.02.2018
(73) Titulaire: Electroducer, 38240 Meylan (FR)
(72) Inventeur: Faurie, Benjamin, 38100 Grenoble (FR)
(74) Mandataire: Nony
(86) Numéro de dépôt international: PCT/EP2016/057385
(87) Numéro de publication internationale: WO 2016/162315

(56) Documents cités:
- US-A1- 2006 241 704
- US-A1- 2009 270 941
- US-A1- 2009 299 443
- US-A1- 2009 318 993
- US-A1- 2010 022 823
- US-A1- 2011 040 344
- US-A1- 2011 251 683
- US-A1- 2012 130 220

## Description

### Domaine technique

La présente invention concerne un ensemble de remplacement d'une valve cardiaque par voie percutanée, comprenant un cathéter de délivrance de valve et le cas échéant un dispositif d'introduction, couramment appelé « introducteur ».

La présente invention a trait plus particulièrement à l'amélioration de l'assistance au remplacement par sidération cardiaque au moyen d'un stimulateur cardiaque.

Bien que décrit en référence au remplacement d'une valve aortique, l'ensemble selon l'invention peut être utilisé en tant qu'ensemble nécessitant ou non la pose d'une prothèse couramment appelée « stent », en particulier en situation d'urgence ou même encore dans des procédures d'intervention complexe.

De même, bien que décrit en référence au remplacement d'une valve aortique, l'ensemble selon l'invention peut tout aussi bien s'appliquer pour le remplacement d'une autre valve du cœur, telle que la valve triscupide ou la valve mitrale.

De manière générale, l'introducteur et/ou le cathéter de délivrance de l'ensemble selon l'invention peut être implanté par voie percutanée au sein d'un patient, et plus précisément, par voie trans-fémorale, par voie trans-aortique, par voie carotidienne ou par voie dite sous-clavière.

### Etat de la technique

Une maladie cardiaque largement connue est celle liée au rétrécissement par calcification de la valve triscupide ou de la valve aortique cardiaque, cette dernière étant la valve qui sépare une cavité cardiaque, à savoir le ventricule gauche de l'aorte et qui permet en position ouverte de laisser passer le sang du cœur vers le reste de l'organisme d'un être humain.

Un rétrécissement serré ou très serré empêche la valve aortique de s'ouvrir normalement et donc génère la maladie aussi appelée sténose aortique calcifiée.

Le traitement de cette maladie consiste à remplacer la valve aortique cardiaque défectueuse.

Le remplacement d'une valve aortique cardiaque défectueuse est le plus fréquemment, réalisé par ouverture du thorax, mise du patient sous circulation extracorporelle, arrêt cardiaque temporaire, et ouverture du coeur, en vue de l'exérèse et du remplacement de la valve native par une valve artificielle ou de prothèse.

Ces étapes successives de l'opération ont pour inconvénients majeurs d'impliquer une hospitalisation relativement longue du patient, d'être complexe et coûteuse et de n'être réservée qu'à une partie des patients atteints, car dans bon nombre de cas, le(s) médecin(s) et/ou chirurgien(s) considèrent que l'intervention chirurgicale dite « à cœur ouvert » ne peut être pratiquée car trop risquée compte tenu de l'état général du patient, notamment du fait de l'arrêt du cœur nécessaire et de la circulation extracorporelle afférente.

Pour remédier à cet inconvénient, il a été envisagé de remplacer une valve cardiaque par voie peu invasive mais toujours sous circulation extracorporelle. On peut citer ici les demandes de brevet internationales WO 93/01768 et WO 97/28807, ainsi que les brevets américains US 5814097, US 5370685 ou US 5545214 qui illustrent des techniques peu invasives connues ainsi que des instruments de mise en oeuvre de ces techniques.

Les techniques existantes ont toutefois été considérées comme n'étant pas parfaitement satisfaisantes et comme susceptibles d'être améliorées.

En particulier, ces techniques ont comme inconvénients majeurs :
- d'imposer en tout état de cause la mise du patient sous circulation extracorporelle; elles sont difficiles à mettre en pratique;
- de ne pas permettre un contrôle précis du diamètre selon lequel la valve native est coupée, en vue du calibrage ultérieur de la valve prothétique;
- d'entraîner des risques de diffusion de fragments de valve native, souvent calcifiée, dans l'organisme, qui peuvent conduire à une embolie,
- des risques de perforation de la paroi aortique ou cardiaque ;
- des risques de reflux aigus du sang lors de l'ablation de la valve native.

Pour pallier les insuffisances de ces techniques, une approche a été la mise en place de valves aortiques artificielles, dites percutanées qui s'inspire des techniques de traitemement endovasculaire par introduction d'un cathéter à l'intérieur d'un vaisseau sanguin, tel que l'aorte.

Ainsi, la valve aortique cardiaque native qui est déficiente par calcification est remplacée par une valve artificielle en évitant l'intervention habituelle de chirurgie cardiaque lourde comme évoqué ci-avant.

La mise en place d'une valve artificielle peut être actuellement réalisée par différentes voies percutanées : par voie trans-fémorale, c'est-à-dire par introduction depuis l'artère fémorale jusqu'au cœur ou par voie trans-apicale, ou par voie trans-aortique, ou par voie carotidienne ou encore par voie sous-clavière, c'est-à-dire toute voie ne nécessitant ni opération à cœur ouvert par l'intermédiaire d'une ouverture du thorax ni circulation extracorporelle.

L'opération en elle-même consiste à déposer une valve artificielle (prothèse), qui reproduit la forme générale d'une valve aortique native normale, au niveau de la valve aortique native calcifiée (malade), cette dernière étant laissée en place et écrasée par la prothèse.

Pour ce faire, la valve artificielle faite en péricarde, membrane fine entourant le cœur, d'origine porcine ou bovine, est fixée préalablement à l'intérieur d'un grillage métallique tubulaire extensible radialement (« stent » en anglais) et constitué par assemblage de fils en matériau à mémoire de forme, par exemple en alliage nickel-titane ou en cobalt-chrome, acier inoxydable 316L pour les stents coronaires.

Puis l'ensemble valve-grillage est comprimé à l'extrémité d'une gaine tubulaire, appelé cathéter de délivrance, qui peut être introduit soit directement dans une artère, soit à l'intérieur d'un introducteur permettant d'accéder à l'artère tout en maintenant une hémostase.

Un médecin interventionniste fait alors coulisser l'ensemble valve-grillage dans l'introducteur ou directement dans le cathéter de délivrance jusqu'à ce que ledit ensemble parvienne à la valve aortique malade. L'ensemble valve-grillage est ensuite déposé au niveau de la valve malade par dilatation d'un ballonnet avant la mise en place.

Il existe aussi des cathéters de délivrance de valve comprenant un ensemble valve-grillage sans ballonnet dans lesquels la valve est auto-expansible qui permet un dépôt de la valve qui s'expanse radialement par simple retrait de la gaine qui l'entoure et donc sans avoir à dilater un ballonnet au préalable.

On pourra se reporter pour plus de précisions aux brevets américains US 7018406, US 7892281, US8652202 et US 8747459. US 2011/251683 A1 divulgue un ensemble de remplacement d'une valve cardiaque par voie percutanée, comprenant un dispositif formant un introducteur comprenant au moins une gaine tubulaire d'introduction, destinée à être introduite dans une artère d'un corps humain et à laisser passer un dispositif d'intervention chirurgicale, tel qu'un cathéter de délivrance.

Lors de la pose à proprement parler il est nécessaire d'effectuer pendant une courte période, un arrêt du cœur temporaire par stimulation ventriculaire rapide pour minimiser le flux transvalvulaire, i.e. entre valvules, et pour éviter à tout le moins réduire l'embolisation potentielle.

Cet arrêt du cœur temporaire aussi appelé couramment « sidération cardiaque » consiste ainsi à faire battre le cœur volontairement à 150 à 200 battements par minute de sorte qu'il n'y ait plus de contraction efficace, ce qui provoque une chute des pressions et simule une tachycardie ou fibrillation ventriculaire et donc une stabilisation du cœur.

Cette stabilisation du cœur permet la stabilisation du ballon et donc d'augmenter la précision de la mise en place de la valve artificielle en quelques secondes.

Il existe des cathéters de stimulation bipolaire, à deux électrodes, dits sondes d'entrainement ou de stimulation électro-systolique, pour la stimulation endocardiaque temporaire du ventricule droit.

Ces sondes de stimulation électro-systolique présentent un certain nombre d'inconvénients détaillés comme suit.

Tout d'abord, une telle sonde constitue un abord veineux central avec un risque de complication vasculaire surajouté pour la population de patients visée qui est fragile. Le registre français désigne « France 2 » qui répertorie les interventions de remplacement de valve aortique, couramment désignées sous l'acronyme anglais TAVI pour «Transcatheter Aortic Valve Implantation », a indiqué comme résultat un taux de risque de complications vasculaires importantes égal à 4,7%. Ce résultat est reporté en page 1709 de la publication [1].

Ensuite, cette sonde est relativement rigide, et de ce fait sa mise en place dans le ventricule droit qui est fragile et dont la paroi est plus fine que celle du ventricule gauche, induit un risque conséquent du phénomène bien connu des médecins interventionnistes sous le terme « tamponnade », qui traduit une insuffisance circulatoire importante pouvant aller jusqu'à la mortalité du patient.

Il est à noter d'ailleurs que ce risque existe aussi bien pendant l'intervention, c'est-à-dire lors de la mise en place de la sonde électro-systolique mais aussi en postopératoire du fait de la mobilisation des patients dans leur lit et donc de la sonde encore présente qui peut alors transpercer la paroi du ventricule droit.

De plus, il y a un risque de déplacement de la sonde de stimulation électro-systolique pendant le moment crucial de la mise en place de la valve. En effet, une sonde de stimulation n'est pas fixée dans une paroi du cœur et peut donc se déplacer et ainsi engendrer une perte de capture du signal électrique de stimulation.

Le cœur n'est alors plus stimulé et donc a des mouvements importants qui gênent le placement de la valve ou du ballon.

Un autre risque lié à l'utilisation de telles sondes est le risque d'infection au site de ponction. Le registre France 2 a indiqué un taux inférieur à 1% : voir publication [1].

Enfin, un médecin interventionniste considère comme non négligeable le temps opératoire supplémentaire lié à la mise en place d'une sonde de stimulation temporaire, qui est une opération pas toujours simple à réaliser.

La publication [2] met en avant les avantages de réaliser cette stimulation ventriculaire sur le ventricule gauche et non pas sur le ventricule droit et de le faire non pas au moyen d'un cathéter de stimulation spécifique par voie transveineuse, mais en mettant en oeuvre un stimulateur cardiaque externe avec le guide-fil utilisé pour les interventions de ce type.

Ainsi, la mise en oeuvre préconisée que l'on retrouve décrite dans cette publication [2] consiste à se servir du guide-fil supportant le ballon d'expansion du stent et introduit dans le ventricule gauche, en tant que partie reliée à la cathode d'un stimulateur cardiaque et d'une électrode cutanée ou aiguille plantée dans le tissu sous cutané, en tant que support de l'anode du stimulateur cardiaque.

Les publications [3] et [4] valident dans le cas d'une intervention par angioplastie coronaire sur une population porcine, l'efficacité d'une stimulation cardiaque temporaire avec une tension électrique de stimulation moindre, par la mise en oeuvre du guide-fil supportant le ballon d'expansion du stent, en tant que partie reliée à la cathode d'un stimulateur cardiaque et d'une électrode cutanée ou aiguille plantée dans le tissu sous cutané, en tant que support de l'anode du stimulateur cardiaque.

Ainsi, ces mises en œuvre préconisées ont pour avantages d'éviter d'avoir à implanter un cathéter supplémentaire dédié, d'éviter un accès supplémentaire au cœur, de réduire le temps et le coût de l'opération, mais aussi de diminuer le taux de complications liées à l'implantation du cathéter dédié, et ce tout en permettant une stimulation aussi efficace que par le biais d'une stimulation par voie transveineuse.

En outre, comparativement aux sondes de stimulation électro-systolique du ventricule droit qui induisent le risque de tamponnade comme explicité ci-avant, le guide-fil utilisé pour cette technique est très stable et vient en appui permanent contre la paroi du ventricule gauche relativement épaisse puisqu'il sert de rail pour avancer l'ensemble stent-ballon-valve au travers de la valve.

Cela étant, cette technique nécessite tout de même la mise en place d'une électrode ou d'une aiguille sous-cutanée supplémentaire qui doit être précise, la mise en place et le maintien de pinces de connexion, de type crocodile sur deux supports éloignés.

Ainsi, il existe encore un besoin d'améliorer l'opération de remplacement d'une valve cardiaque nécessitant une sidération du cœur temporaire lors de la pose de la valve artificielle, notamment afin de rendre plus aisée et plus rapide la mise en place et la manipulation des électrodes de stimulateur cardiaque pour le(s) chirurgien(s) en charge de l'opération.

Le but de l'invention est de répondre au moins en partie à ce besoin.

### Exposé de l'invention

Pour ce faire, l'invention a pour objet selon une première alternative un ensemble de remplacement d'une valve cardiaque par voie percutanée, comprenant :
- un dispositif formant un introducteur comprenant au moins une gaine tubulaire d'introduction, destinée à être introduite dans une artère d'un corps humain et à laisser passer un dispositif d'intervention chirurgicale, tel qu'un cathéter de délivrance de valve, et au moins un élément conducteur électrique dont une portion distale est apparente sur une au moins une partie de la périphérie extérieure de la gaine de sorte à être en contact avec le tissu sous-cutané du corps ou avec l'artère et dont une portion proximale est accessible depuis l'extérieur du corps de sorte à servir de connexion à une électrode d'un stimulateur cardiaque ;
- au moins un guide-fil destiné à être introduit dans la gaine tubulaire de l'introducteur pour l'avancement d'une valve artificielle destinée à remplacer la valve cardiaque, le guide-fil comprenant une partie métallique servant en outre de connexion à l'autre électrode du stimulateur cardiaque.

Selon une deuxième alternative, l'invention a pour objet un ensemble de remplacement d'une valve cardiaque par voie percutanée, comprenant :
- un dispositif formant un cathéter de délivrance de valve comprenant au moins une gaine tubulaire d'introduction, destinée à être introduit dans une artère d'un corps humain, et au moins un élément conducteur électrique dont une portion distale est apparente sur une au moins une partie de la périphérie extérieure de la gaine de sorte à être en contact avec le tissu sous-cutané du corps ou avec l'artère et dont une portion proximale est accessible depuis l'extérieur du corps de sorte à servir de connexion à une électrode d'un stimulateur cardiaque ;
- au moins un guide-fil destiné à être introduit dans la gaine tubulaire du cathéter de délivrance pour l'avancement d'une valve artificielle destinée à remplacer la valve cardiaque, le guide-fil comprenant au moins une partie métallique servant en outre de connexion à l'autre électrode du stimulateur cardiaque.

Selon un mode de réalisation, l'électrode du stimulateur cardiaque connectée à l'élément conducteur électrique de l'introducteur ou du cathéter de délivrance étant l'anode tandis que celle connectée à la partie métallique du guide-fil est la cathode.

Ainsi, l'invention consiste essentiellement en l'intégration du support métallique d'une électrode du stimulateur cardiaque directement dans la gaine d'introduction dans l'artère d'un patient.

Cette gaine d'introduction peut être celle d'un introducteur ou directement celle d'un cathéter de délivrance, de taille réduite par rapport à celle d'un introducteur. Un cathéter de délivrance de valve ne nécessite pas de facto d'introducteur puisqu'il peut être introduit directement dans l'artère d'un patient.

Autrement dit, on ajoute une fonction de support de stimulation cardiaque à un introducteur ou à un cathéter de délivrance dont la fonction de base est de pénétrer à l'intérieur d'une artère d'un patient pour laisser passer un ensemble valve-stent-ballon ou un ensemble valve auto-expansible-stent pour le remplacement d'une valve aortique déficiente.

Grâce à l'invention, il n'est plus nécessaire de planter une aiguille dans les tissus sous-cutanés ou une électrode cutanée pour servir de support à l'électrode, typiquement l'anode d'un stimulateur cardiaque.

Il n'est pas non plus nécessaire d'utiliser et de mettre en place une sonde électro-systolique comme selon l'état de l'art, appelée couramment sonde temporaire.

De plus, grâce à l'invention, l'intensité de stimulation nécessaire à la sidération cardiaque est plus faible que dans les solutions selon l'état de l'art, du fait d'une impédance plus faible du système vasculaire par rapport au tissu sous-cutané.

L'élément conducteur électrique est avantageusement un fil ou une bande métallique logé(e) au moins en partie dans l'épaisseur de la gaine dont une portion distale est apparente à la périphérie extérieure de la gaine.

Le(s) chirurgien(s) en charge de l'opération peu(ven)t ainsi relier l'électrode, typiquement l'anode du stimulateur cardiaque à une portion proximale de fil ou bande métallique en périphérie de l'introducteur ou du cathéter de délivrance facilement puis relier comme usuellement l'autre électrode, typiquement la cathode au guide-fil de l'ensemble valve-stent-ballon ou valve auto-expansible-stent.

Dans un ensemble de l'invention, on prévoit qu'une autre portion de fil ou bande métallique, distale de celle sur laquelle se fait la connexion électrique, à l'extérieur du corps de l'être humain opéré, soit apparente, c'est-à-dire non recouverte par la gaine tubulaire d'introduction, pour être directement en contact avec les tissus sous-cutanés du corps ou avec l'espace vasculaire.

Ainsi, l'étape de préparation à l'arrêt du cœur est plus simple et plus rapide à réaliser.

En plus, l'inventeur pense qu'un introducteur selon l'invention ou un cathéter de délivrance selon l'invention peut diminuer les risques de complications liées aux sondes de stimulation électro-systolique selon l'état de l'art qui sont placées dans le ventricule droit.

L'introducteur ou le cathéter de délivrance selon l'invention peut être introduit par voie transapicale ou par voie transfémorale, qui est privilégiée pour son caractère moins invasif pour des patients plus fragiles.

L'introducteur ou le cathéter de délivrance selon l'invention peut intégrer un système de perfusion périphérique, usuellement appelé « Flush », qui peut être mis en place pour nettoyer l'intérieur de l'introducteur ou du cathéter de tout caillot de sang susceptible d'être présent.

L'introducteur ou le cathéter de délivrance selon l'invention peut tout à fait être réalisé sur la base d'un introducteur respectivement d'un cathéter de délivrance déjà existant, seule une étape de fabrication supplémentaire qui consiste à loger le fil ou bande métallique à l'intérieur de la gaine d'introduction et à laisser apparentes les portions proximale et distale du fil (bande) métallique, doit être prévue.

Ainsi, avantageusement, pour un ensemble de remplacement de valve aortique cardiaque, l'introducteur peut être réalisé sur la base des mêmes dimensions, formes et matériaux qu'un introducteur connu, par exemple celui sous la dénomination commerciale *« ensemble de gaine d'introduction eSheath d'Edwards* » commercialisé par la société Edwards Lifesciences.

La valve artificielle peut être introduite et positionnée dans l'artère au moyen d'un cathéter de délivrance selon l'invention ou au moyen d'un cathéter de valve classique, lui-même introduit dans l'introducteur. La valve artificielle occupe alors une position repliée et ne fait pas obstacle à l'introduction et coulissement du cathéter de valve dans l'introducteur puis dans l'artère ou dans le cathéter de délivrance selon l'invention puis dans l'artère.

Puis, en position déployée, la valve artificielle vient prendre appui contre la paroi extérieure de la valve cardiaque native en lieu et place de cette dernière en l'écrasant.

Un cathéter de valve classique ou selon l'invention permet ainsi d'introduire et de positionner la valve artificielle à l'endroit adéquat, par le même geste d'opération que celui permettant d'ouvrir et d'écraser la valve native. Après ouverture et écrasement de cette dernière, le cathéter de valve est coulissé axialement dans la direction distale afin d'amener la valve artificielle au niveau adéquat dans l'ouverture de la valve native.

Le(s) chirurgien(s) intervenant sur le patient applique(nt) pendant l'ouverture et l'écrasement de la valve native puis après, une stimulation cardiaque au moyen du stimulateur cardiaque extérieur, le courant électrique circulant entre la cathode et l'anode du stimulateur, la cathode étant reliée au guide-fil de la valve artificielle et l'anode étant reliée au fil ou à la bande métallique intégrée dans la gaine tubulaire extérieure de l'introducteur ou du cathéter de délivrance selon l'invention.

Simultanément à la stimulation ventriculaire, la valve artificielle est déployée. Le cathéter de valve est ensuite retiré.

En résumé, les avantages d'un ensemble selon l'invention comparativement à ceux selon l'état de l'art, tels que présentés en préambule, sont nombreux, parmi lesquels on peut citer :
- une mise en place plus simple et plus rapide d'une électrode, typiquement l'anode de stimulation ventriculaire lors de l'opération de remplacement d'une valve aortique déficiente ;
- la suppression de la nécessité de planter une aiguille sous-cutanée supplémentaire en tant que support d'électrode, typiquement d'anode de stimulateur cardiaque ;
- un temps et coût moindres de l'opération de remplacement d'une valve cardiaque déficiente ;
- une efficacité accrue de la stimulation temporaire en vue de réaliser la sidération cardiaque souhaitée du fait de la moindre impédance du système vasculaire rencontré par le courant électrique de stimulation puisque le fil ou la bande métallique intégré à l'introducteur ou à un cathéter de délivrance est directement en contact avec ledit système, au contraire des aiguilles selon l'état de l'art qui viennent en contact avec le tissu cutané d'un patient qui présente nécessairement une impédance plus élevée;
- une efficacité accrue de la stimulation temporaire en vue de réaliser la sidération cardiaque souhaitée du fait de la stabilité du guide rigide (diamètre de l'ordre de 1,455mm) dans le ventricule gauche, au lieu de l'instabilité de la sonde électro-systolique selon l'état de l'art mise en place dans le ventricule droit ;
- la possibilité de réaliser la stimulation temporaire cardiaque avec un courant électrique moins élevé du fait de l'impédance moindre du système vasculaire rencontré entre les deux électrodes du stimulateur extérieur ;
- la suppression des risques de complication liés aux sondes de stimulation temporaires selon l'état de l'art qui sont placées dans le ventricule droit ;
- l'utilisation possible de l'introducteur ou du cathéter de délivrance pour plusieurs types d'interventions TAVI différentes, telles qu'un remplacement de valve aortique, pulmonaire, tricuspide ou mitrale. En particulier, pour le remplacement d'une valve tricuspide dégénérée, seule la technique d'introduction d'une sonde de stimulation dans le ventricule droit au moyen du guide-rail (diamètre 0,89 mm) est envisageable car il n'est pas envisageable de mettre à la fois le guide-rail et une sonde électro-systolique puisque l'expansion du ballon ou de la prothèse valvulaire viendrait comprimer la sonde avec le risque inhérent d'interruption de la stimulation ou de coincer la sonde de stimulation;
- l'utilisation possible dans la population pédiatrique lors des procédures valvulaires ou cardiaques sur des cœurs plus tachycardes, plus mobiles que dans la population adulte. De plus, il s'agit d'une population chez laquelle la ponction veineuse fémorale peut être très difficile ainsi que la mise en place d'une sonde de stimulation ventriculaire droite. Enfin, les parois ventriculaires droites infantiles sont fines et fragiles majorant ainsi le risque de complication grave telle que la tamponnade. Il s'agit d'ailleurs de la population décrite dans la publication [2] ;
- l'utilisation possible dans le domaine d'angioplastie coronaire dans l'urgence et dans les procédures complexes, dans lequel une stimulation cardiaque temporaire doit être réalisée de manière efficace et très rapidement. A cet effet, un introducteur ou un cathéter de délivrance selon l'invention évite le temps de mise en place d'une électrode ou d'une aiguille sous-cutanée supplémentaire comme selon l'état de l'art, ce qui peut être déterminant lors de ces interventions.

Selon un mode de réalisation avantageux, l'introducteur ou le cathéter de délivrance selon l'invention comprend :
- une échancrure distale réalisée dans l'épaisseur de la gaine tubulaire et laissant apparente la portion distale de fil ou de bande métallique de sorte à réaliser le contact avec une paroi du système vasculaire du corps ;
- une échancrure proximale, réalisée dans une zone proximale de l'introducteur destinée à être à l'extérieur du corps, et laissant apparente la portion proximale de fil ou de bande métallique de sorte à réaliser la connexion avec l'électrode du stimulateur cardiaque.

Selon une variante de réalisation avantageuse selon laquelle l'introducteur ou le cathéter de délivrance comprend un dispositif de rinçage à robinets, dit « flush », pour rincer l'intérieur de l'introducteur au moyen d'un liquide de rinçage approprié, la portion proximale du fil ou de la bande métallique est apparente au niveau du flush. Cette variante est avantageuse car elle simplifie encore la mise en place de la stimulation temporaire puisque le flush est directement orienté du côté du stimulateur cardiaque extérieur. Et donc, la mise en place et le maintien de la pince de connexion peuvent se faire du côté du stimulateur extérieur. Il est donc plus aisé pour médecin interventionniste de contrôler à la fois le maintien de la pince de connexion et le stimulateur cardiaque extérieur. La portion proximale du fil au niveau du flush peut être conformé en un connecteur électrique sur lequel il est possible de venir directement un connecteur complémentaire relié directement au stimulateur cardiaque externe.

De préférence, la échancrure proximale et la portion proximale de fil ou bande métallique sont conformées pour permettre le pincement d'une pince de type crocodile sur ladite portion. On peut ainsi utiliser ces pinces conventionnelles sans avoir à utiliser des pinces spécialement conçues pour l'introducteur selon l'invention.

De préférence encore, la portion distale du fil ou bande métallique est agencée :
- soit à une distance D, mesurée depuis l'extrémité proximale de l'introducteur, comprise entre la moitié et les trois quart de la hauteur de la portion tubulaire de la gaine définissant la zone proximale (Z_{E}) de l'introducteur ;
- soit à une distance comprise entre 20 et 60 cm de la zone distale du cathéter de délivrance.

Pour l'alternative avec le cathéter de valve, une zone de contact électrique à une distance entre 20 et 60 cm de la zone distale du cathéter permet avantageusement de s'assurer du contact au niveau de l'endothélium intra-vasculaire aortique.

La section du fil ou de la bande métallique est avantageusement comprise entre 0,25 et 5 mm².

Selon un autre mode de réalisation avantageux de l'invention, l'élément conducteur électrique comprend un revêtement conducteur électrique, tel qu'un revêtement en carbone, déposé sur la périphérie extérieure de la gaine.

Selon ce mode, la gaine est avantageusement un revêtement bi-couche avec une couche interne isolante électrique et le revêtement conducteur électrique déposé sur la couche interne.

De préférence, le revêtement conducteur électrique est déposé sur toute la hauteur de la périphérie extérieure de la gaine à l'exception de l'extrémité distale de cette dernière. On s'assure ainsi du contact à coup sûr entre le revêtement conducteur électrique et le tissu sous-cutané du corps ou la paroi de l'artère fémorale du patient opéré dans lequel est introduit l'introducteur ou le cathéter de délivrance selon l'invention

Selon une variante particulièrement avantageuse, l'ensemble comprend :
- un embout intégrant en son sein une bague dans laquelle la gaine est emmanchée à force,
- une languette de connexion en matériau conducteur électrique, dont une extrémité est insérée à travers la bague de sorte à établir un contact électrique avec le revêtement électrique conducteur, et dont l'autre extrémité fait saillie à l'extérieur de l'embout, la surface de cette extrémité à l'extérieur de l'embout étant adaptée pour être serrée par une pince de connexion destinée à être reliée à une électrode d'un stimulateur cardiaque. La réalisation de l'ensemble selon cette variante est particulièrement efficace car la connexion électrique avec le stimulateur cardiaque externe se fait aisément à l'aide d'une pince qui peut être conçue spécifiquement pour venir se pincer sur la languette de connexion. Cela rend fiable la connexion électrique et permet à un interventionniste de réaliser la connexion facilement et rapidement.

L'invention a également pour objet un guide-fil pour un ensemble selon l'invention, tel que décrit ci-dessus, comprenant une âme métallique revêtue d'un revêtement isolant électrique sur une portion centrale de longueur L1, entre l'extrémité proximale et l'extrémité distale du guide-fil, l'âme métallique étant non isolée électriquement sur le reste de la longueur du guide fil.

Avantageusement, l'extrémité distale non isolée électriquement, destinée à venir en contact avec la paroi du ventricule gauche du cœur d'un patient, est une portion plus souple que le reste du guide-fil.

Typiquement pour un guide-fil d'une longueur totale L de l'ordre de 260 cm, la longueur L2 de l'âme métallique non revêtue à l'extrémité distale et donc non isolée électriquement est de l'ordre de 6 à 10 cm, la longueur L1 du revêtement isolant électrique est de l'ordre de 110 cm, la longueur de l'âme métallique L3 égale à L-(L1+L2) non revêtue à l'extrémité proximale étant de l'ordre de 140 à 144 cm.

En gardant la périphérie du guide-fil isolante uniquement sur la longueur L mais non isolante aux extrémités distale et radiale, on permet une stimulation cardiaque d'urgence même en cas de retrait du cathéter de délivrance. En effet, en situation d'urgence cardiaque, l'âme métallique peut toujours servir de connexion à une électrode, typiquement la cathode de stimulateur cardiaque, et il est toujours possible de planter une aiguille dans les tissus sous-cutanés ou de laisser l'introducteur selon l'invention en place, pour servir de support à l'autre électrode, typiquement l'anode du stimulateur cardiaque. L'isolation électrique entre anode et cathode est alors réalisée par le revêtement isolant du guide-fil.

Les avantages d'un guide-fil conforme à l'invention sont nombreux parmi lesquels on peut citer :
- éviter l'utilisation en urgence d'une sonde d'entraînement électro-systolique dont l'efficacité de stimulation n'est même pas garantie à 100%, en particulier du fait des difficultés de sa mise en place dans un temps contraint ;
- assurer l'efficacité de la stimulation qui n'est pas garantie avec un guide-fil selon l'état de l'art, typiquement un guide-fil métallique avec un revêtement en téflon, qui n'est pas isolé électriquement. L'inventeur a ainsi pu constater quelques échecs de stimulation lorsqu'un cathéter ou ballonnet selon l'état de l'art n'isole plus le guide-fil ;
- permettre la mise en place d'un stimulateur cardiaque extérieur et sa sonde d'entraînement avec tranquillité et sans caractère d'urgence puisque la stimulation est assurée de fait entre le guide-fil selon l'invention et l'introducteur ou le cathéter de délivrance selon l'invention.

L'invention a encore pour objet l'utilisation de l'ensemble avec introducteur ou cathéter de délivrance décrit précédemment pour le remplacement d'une valve aortique, pulmonaire, tricuspide ou mitrale.

### Description détaillée

D'autres avantages et caractéristiques de l'invention ressortiront mieux à la lecture de la description détaillée de l'invention faite à titre illustratif et non limitatif en référence aux figures suivantes parmi lesquelles :
- la figure 1 est une vue en perspective d'un introducteur selon l'état de l'art, destiné à être introduit dans une artère fémorale au niveau de l'aine d'un patient ;
- les figures 2A à 2C montrent en vue de coupe longitudinale partielle différentes étapes de coulissement d'un cathéter de valve dans l'introducteur selon la figure 1, afin de réaliser le placement d'une valve artificielle en lieu et place d'une valve aortique native déficient ;
- la figure 3 montre en vue schématique en perspective depuis l'extérieur d'un patient à la fois l'étape de mise en place d'un cathéter de valve et des électrodes de stimulation cardiaque selon l'état de l'art ;
- la figure 4 est une vue schématique en perspective d'un cathéter de délivrance selon l'état de l'art, destiné à être introduit directement dans l'artère d'un patient sans nécessité d'un introducteur ;
- la figure 5 est une vue en perspective d'un dispositif formant introducteur selon l'invention ;
- la figure 6 est une vue de coupe longitudinale partielle de la gaine tubulaire de l'introducteur selon la figure 5, montrant l'intégration d'une bande métallique dans la gaine conformément à l'invention ;
- la figure 7 illustre l'utilisation d'un ensemble selon l'invention avec un cathéter selon l'invention introduit directement dans une artère d'un patient, la délivrance de la valve prothétique depuis le cathéter s'effectuant via l'arche aortique ;
- la figure 8 est une vue schématique d'un guide-fil selon une variante avantageuse de l'invention ;
- la figure 9 est une vue en coupe longitudinal d'un mode de réalisation avantageux d'un dispositif formant introducteur selon l'invention ;
- les figures 9A et 9B sont des vues de détail selon A et selon B respectivement de l'extrémité distale et proximale de l'introducteur selon la figure 9 ;
- la figure 10 est vue de face d'une pince de connexion électrique particulièrement adaptée à la connexion avec l'introducteur de l'invention selon les figures 9 et 9B ;
- la figure 10A est une vue en coupe longitudinale selon A-A de la pince selon la figure 10 ;
- les figures 11A et 11B sont des vues en perspective de la pince selon la figure 10, montrant respectivement la pince dans sa position fermée et dans sa position ouverte ;
- la figure 12 est une vue en perspective du clip à mâchoires en matériau conducteur électrique, qui est intégré dans la pince de la figure 10 et qui est destiné à être serti sur la partie de connexion de l'introducteur selon l'invention afin de réaliser la connexion électrique.

Dans la description qui va suivre ainsi que dans l'ensemble de la présente demande, les termes « distal » et « proximal » sont utilisés par référence avec le corps d'un patient dont la valve aortique native déficiente est remplacée par une valve aortique artificielle. Ainsi, l'extrémité distale d'un introducteur est l'extrémité située le plus à l'intérieur du patient lors de l'opération de remplacement.

Par souci de simplification, les mêmes éléments dans le dispositif selon l'invention et dans celui selon l'état de l'art sont désignés par les mêmes références.

On précise que les différents éléments ne sont pas nécessairement représentés à l'échelle.

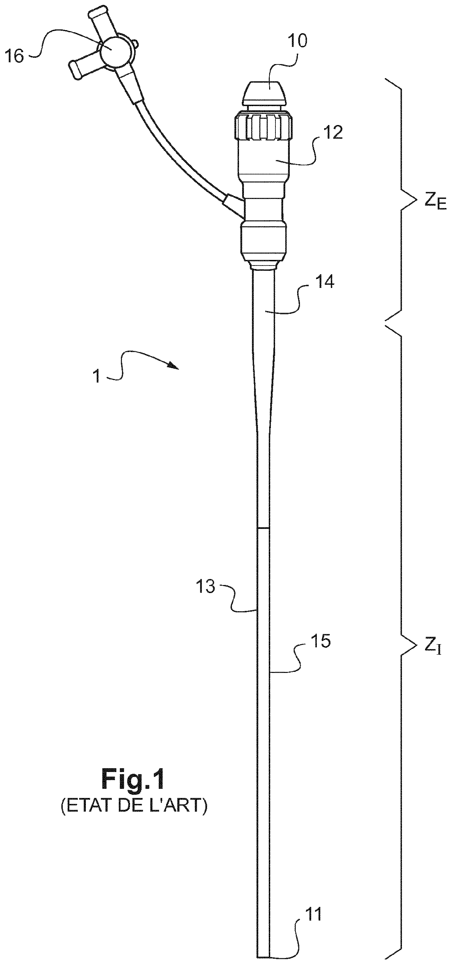

La figure 1 représente un introducteur 1 de remplacement d'une valve cardiaque par voie transfémorale.

Cet introducteur 1 de forme générale tubulaire comprend entre son extrémité proximale 10 et son extrémité distale 11, un embout 12 prolongé par au moins une gaine tubulaire 13 extérieure formée de deux portions tubulaires 14, 15 du côté proximal vers le côté distal, considérés par rapport à l'introduction dans une artère fémorale d'un patient à opérer, c'est-à-dire de haut en bas sur la figure 1.

L'embout 12 intègre en général un ensemble de vannes étanches pour réaliser une hémostase, c'est-à-dire pour assurer le maintien du sang à l'intérieur des vaisseaux sanguins du patient lors de l'intervention.

La gaine tubulaire 13 peut être extensible ou non pour pouvoir laisser passer un dispositif d'intervention chirurgicale tel qu'un cathéter de valve comme expliqué par la suite. Le matériau constitutif de la gaine 13 est un matériau biocompatible, tel qu'en silicone. Elle peut tout aussi bien être réalisée en Teflon™ ou en polyuréthane. La gaine peut être avantageusement recouverte à l'extérieur d'une couche hydrophile et à l'intérieur d'une couche à matériau à faible coefficient de frottement pour faciliter le coulissement d'un dispositif d'intervention.

L'introducteur 1 illustré en figure 1 comprend également un dispositif de rinçage 16 à robinets, couramment appelé « flush », intégré pour rincer l'intérieur de l'introducteur 1 au moyen d'un liquide de rinçage approprié.

Tous les éléments de l'introducteur 1 présents dans la zone proximale ou extérieure Z_{E} sont destinés à rester à l'extérieur du corps du patient, toute la portion distale 15 de la gaine 13 définissant la zone distale Z_{I} est destinée à être introduite dans une artère fémorale d'un patient.

L'introducteur 1 illustré est par exemple celui commercialisé sous la dénomination commerciale « *ensemble de gaine d'introduction eSheath d'Edwards »* commercialisé par la société Edwards Lifesciences.

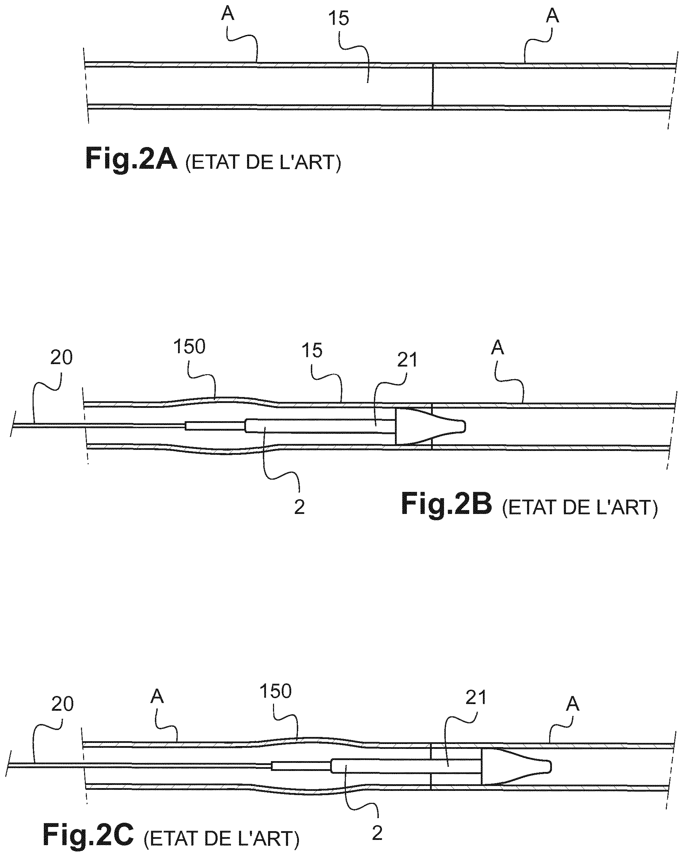

On a représenté aux figures 2A à 2C l'avancement d'un cathéter de valve 2 constitué d'un guide-fil 20 et d'un ensemble 21 constitué d'une valve artificielle fixée à un stent à expansion radiale et d'un ballon gonflable pour réaliser cette expansion, à l'intérieur de la portion distale 14 de la gaine tubulaire de l'introducteur 1 déjà introduit dans une artère fémorale A.

La pointe de l'ensemble 21 permet de pénétrer aisément la valve aortique native déficiente.

On voit sur ces figures qu'au fur et à mesure du coulissement du cathéter de valve 2, la portion 15 de la gaine tubulaire se déforme temporairement radialement en formant une légère excroissance 150. Lorsque la gaine tubulaire n'est pas extensible, elle ne se déforme pas radialement.

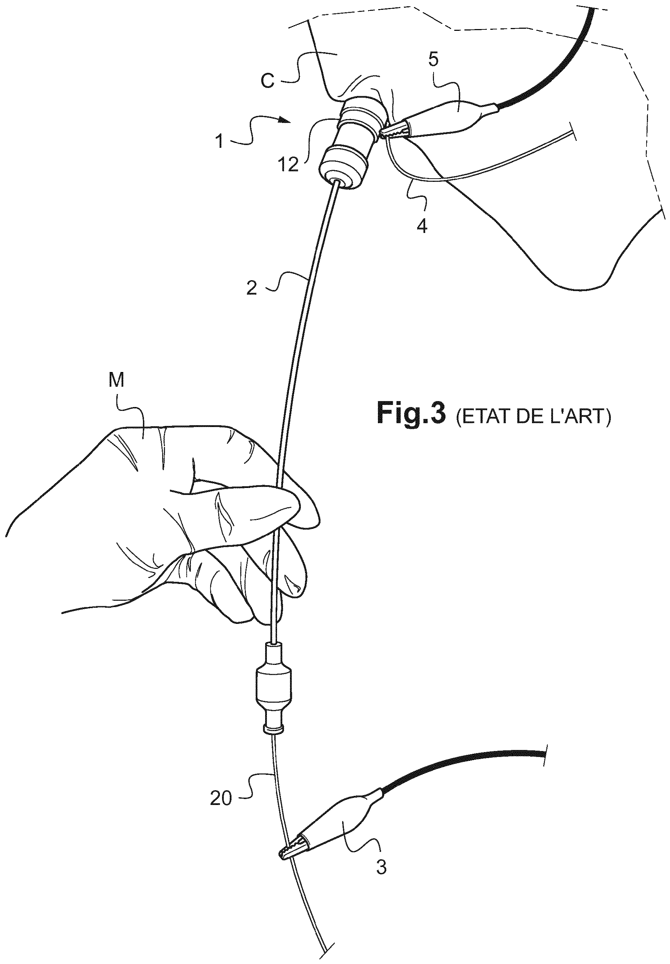

En figure 3, on peut voir que la main M d'un chirurgien réalise l'introduction du cathéter de valve 2 dans l'introducteur 1 déjà introduit dans l'artère fémorale d'un patient, l'embout 12 faisant saillie à l'extérieur du corps C.

Cette introduction du cathéter de valve 2 permet d'amener l'ensemble 21 au niveau de la valve aortique calcifiée déficiente qu'il faut remplacer.

Usuellement, comme visible également sur cette figure 3, une pince 3 connue sous l'appellation pince-crocodile est fixée par pincement sur le guide-fil 20 du cathéter de valve 2. Cette pince 3 est reliée à la cathode d'un stimulateur cardiaque non représenté, situé à l'extérieur du corps C.

Une aiguille non montrée est également plantée dans les tissus sous-cutanés du corps C du patient à opérer. Sur cette aiguille est fixé un fil métallique 4.

Une pince 5 de type crocodile est fixée également par pincement sur le fil métallique 4.

Cette pince 5 est reliée à l'anode du stimulateur cardiaque extérieur au corps.

Ainsi, lorsque la valve artificielle est au niveau de la valve aortique native à remplacer, le chirurgien réalise préalablement à la mise en place proprement dite de la valve artificielle, c'est-à-dire au gonflement du ballon et donc l'expansion du stent auquel est fixée la valve, une stimulation ventriculaire rapide du ventricule gauche.

Pour cela, un signal électrique est délivré entre la cathode et l'anode par le biais des pinces 3, 5, le ballon jouant le rôle d'isolant électrique entre ces deux électrodes.

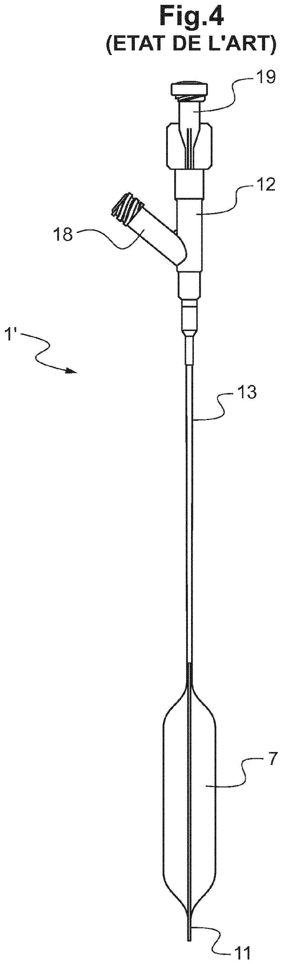

La figure 4 illustre un cathéter de délivrance 1' qui peut être introduit directement dans l'artère d'un patient sans nécessité d'un introducteur. Plus précisément, le cathéter 1' comprend un embout 12 prolongé par une gaine d'introduction 13. L'embout 12 comprend une connexion 18 pour le gonflage/dégonflage d'un ballonnet 7 à l'extrémité distale 11 qui permet d'expandre une valve prothétique non représentée.

Confronté à de nombreuses opérations de remplacement de valve aortique par voie fémorale telle qu'elle vient d'être brièvement décrite, et en particulier à la mise en place précise et délicate de l'aiguille sous-cutanée supplémentaire, ainsi que la mise en place et le maintien des pinces de connexion, de type crocodile sur deux supports éloignés, l'inventeur de la présente invention a pensé intégré le fil métallique 4 directement dans un introducteur 1 ou dans un cathéter de délivrance 1'.

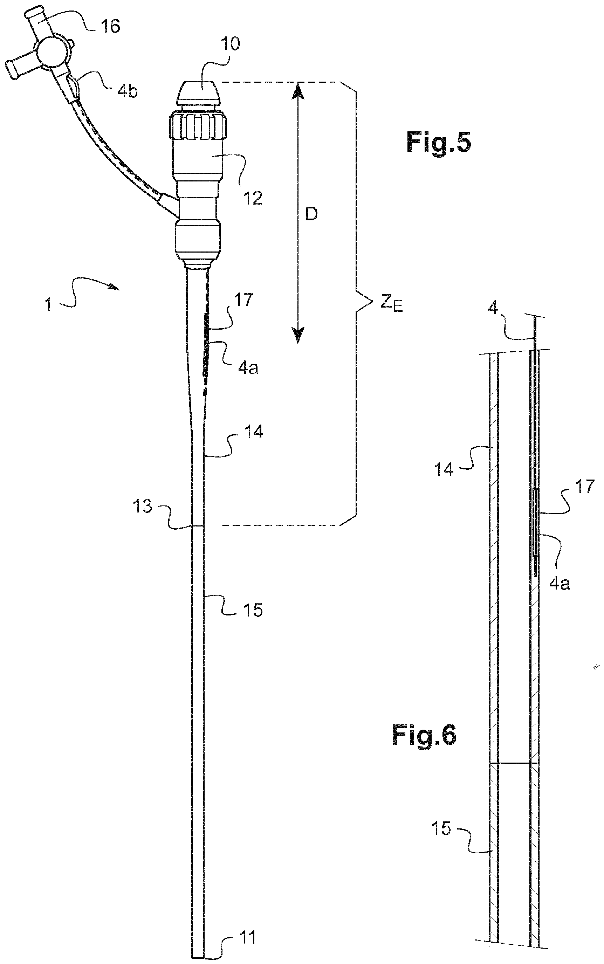

Plus précisément, comme montré aux figures 5 et 6, le fil ou la bande métallique 4 qui sert de connexion à l'anode du stimulateur cardiaque est noyé(e) au moins partiellement dans le matériau isolant électrique et biocompatible de la gaine tubulaire 13.

Le fil ou bande métallique 4 peut être réalisé sous la forme d'une tresse métallique afin de lui conférer des caractéristiques de souplesse nécessaires pour accommoder les déformations de la gaine extensible lors de l'introduction d'un cathéter de valve dans l'introducteur 1.

Le matériau métallique du fil ou bande 4 est choisi pour être également biocompatible, tel que le titane, ou un acier inoxydable recouvert le cas échéant d'une couche à la fois anti-adhésive pour permettre le glissement à l'intérieur de l'artère et conductrice pour assurer la continuité électrique entre le cœur du fil ou de la bande avec le tissu cutané ou la paroi de l'artère en contact.

Une échancrure distale 17 pratiquée dans la portion 15 de la gaine tubulaire laisse apparente une portion distale 4a du fil ou de la bande métallique 4. Cette portion distale 4a vient ainsi directement en contact avec le tissu sous-cutané du corps ou la paroi de l'artère fémorale du patient opéré.

Il va de soi que l'on veille à ce que la portion apparente 4a, 4b du fil ou de la bande métallique ne fasse saillie à l'extérieur de la gaine tubulaire pour ne pas risquer de créer des lésions lors de l'introduction du cathéter 1.

On positionne cette échancrure distale 17 à une distance D de l'extrémité distale 11 telle qu'on s'assure qu'il y ait bien un contact de la portion distale 4a du fil ou bande métallique 4 avec le tissu cutané ou la paroi de l'artère. De préférence encore, la distance D est telle que la portion distale 4a soit apparente dans une zone correspondant à environ la moitié de la longueur de la portion tubulaire 14. On s'assure ainsi qu'il y a un contact avec l'espace vasculaire, ce qui peut contribuer à améliorer l'efficacité de la stimulation cardiaque.

A l'autre extrémité, une échancrure proximale est pratiquée dans une portion de la zone proximale Z_{E} pour rendre apparente une portion proximale 4b du fil ou bande métallique 4 afin que celui-ci soit accessible depuis l'extérieur du corps C.

Au niveau de l'échancrure proximale, le fil ou la bande métallique 4 ainsi que l'échancrure 17 sont conformées de sorte à ce qu'une pince de connexion telle qu'une pince 5 de type crocodile vienne se fixer par pincement sur la portion proximale 4b.

Selon une variante de réalisation avantageuse, illustrée en figure 5, la portion proximale 4b est apparente au niveau du dispositif flush 16.

Cette variante est avantageuse car la mise en place de la pince de connexion 5 est directement faite du côté où se trouve usuellement le stimulateur cardiaque extérieur, ce qui facilite encore l'opération pour un médecin interventionniste.

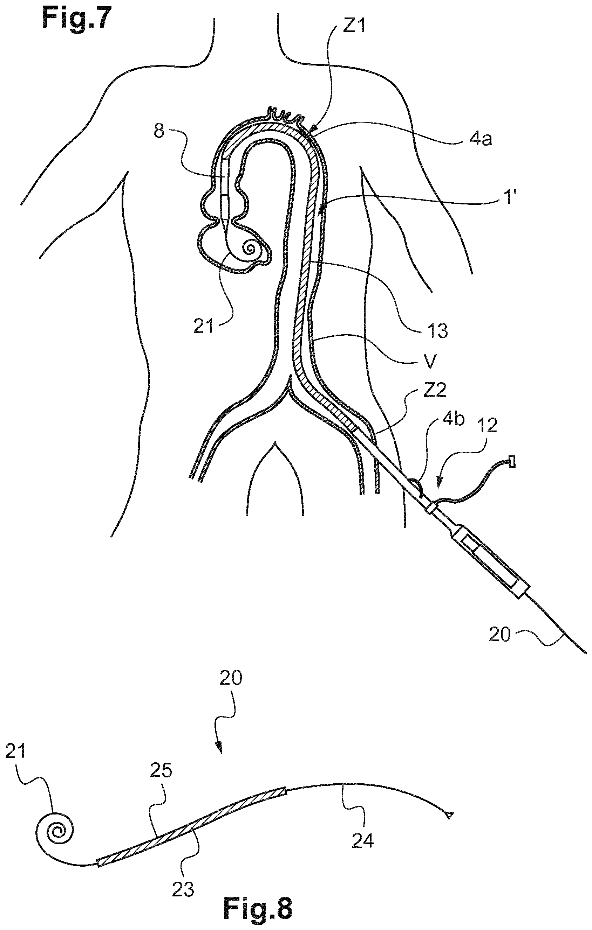

On a représenté en figure 7, l'utilisation d'un ensemble avec cathéter de délivrance 1' selon l'invention pour la délivrance d'une valve prothétique 8 par l'arche aortique d'un patient.

Comme on peut le voir sur cette figure 7, le cathéter 1' introduit depuis l'artère fémorale au niveau de la zone Z2 vient en contact avec l'arche aortique au niveau de la zone Z1. L'emplacement de la portion distale 4a du fil ou bande métallique selon l'invention est judicieusement choisi pour que cette portion 4a vienne toucher la paroi de l'arche aortique dans cette zone Z1. La portion proximale 4b du fil ou bande métallique est quant à elle à l'extérieur du corps, apparente sur une partie de l'embout 12 du cathéter.

On a représenté en figure 8, un guide-fil 20 selon une variante avantageuse de l'invention, qui peut être introduit directement dans un cathéter de délivrance 1' selon l'invention comme montré en figure 7, ou dans un introducteur 1 selon l'invention.

Le guide-fil 20 est constitué d'une âme métallique sur toute sa longueur revêtue d'un revêtement isolant électrique 25 uniquement sur une portion centrale 23 de longueur L1, entre l'extrémité proximale 22 et l'extrémité distale 24 du guide-fil. L'âme métallique ne comprenant pas de revêtement isolant sur sa périphérie à ses extrémités distale 22 et proximale 24, elle est non isolée électriquement sur le reste de la longueur du guide fil.

Avantageusement, l'extrémité distale 22 non isolée électriquement, destinée à venir en contact avec la paroi du ventricule gauche du cœur d'un patient, est une portion plus souple que le reste du guide-fil. Plus précisément, comme montré en figure 7, l'extrémité distale souple 22 s'enroule sur elle-même en venant en contact avec l'endothélium ventriculaire gauche du patient.

Ainsi, en situation d'urgence où il est nécessaire de stimuler le cœur il est toujours possible de planter une aiguille dans les tissus sous-cutanés si le cathéter 1' est retiré ou de laisser l'introducteur selon l'invention en place, pour servir de support à l'autre électrode, typiquement l'anode du stimulateur cardiaque.

Il est alors tout-à-fait possible de stimuler efficacement puisque l'extrémité distale 22 du guide-fil 20 assure toujours le contact électrique et donc le passage du courant depuis la cathode et le revêtement isolant 25 assure parfaitement l'isolation entre l'anode et la cathode sur le trajet intra-vasculaire.

L'invention fournit ainsi un ensemble avec introducteur ou cathéter de délivrance utilisable plus particulièrement lors d'un remplacement d'une valve cardiaque par voie percutanée, permettant de remédier aux inconvénients des techniques antérieures et d'améliorer encore l'efficacité de la stimulation cardiaque temporaire pour réaliser la sidération cardiaque souhaitée.

En effet, le dispositif selon l'invention permet d'éviter la mise en place précise et délicate d'une aiguille sous-cutanée pour la connexion à l'anode d'un stimulateur cardiaque.

Le dispositif selon l'invention permet ainsi un gain de temps pour l'opération de remplacement.

En outre, le dispositif selon l'invention permet d'améliorer l'efficacité intrinsèque de la stimulation temporaire pour atteindre la sidération cardiaque recherchée car l'impédance entre les deux électrodes du stimulateur cardiaque est celle de l'espace vasculaire puisque le fil ou bande métallique vient en contact avec ce dernier lors de l'introduction de la gaine. Du fait de cette impédance moindre que celle des tissus cutanés rencontrée par les sondes de stimulation temporaires selon l'état de l'art, il est possible de faire délivrer un courant électrique de stimulation par le stimulateur extérieur moins important que selon l'état de l'art pour une efficacité au moins aussi bonne.

L'invention n'est pas limitée aux exemples qui viennent d'être décrits ; on peut notamment combiner entre elles des caractéristiques des exemples illustrés au sein de variantes non illustrées.

D'autres variantes et améliorations peuvent être prévues sans pour autant sortir du cadre de l'invention.

Ainsi, si dans les exemples illustrés, les parties qui servent de connexion à un stimulateur et qui sont intégrées soit dans un introducteur soit dans un cathéter de délivrance sont des portions apparentes de fil ou bande métallique, on peut aussi envisager en lieu et place d'avoir un revêtement continu sur la surface de gaine d'introduction qui soit conducteur électrique.

Ce revêtement peut notamment être en carbone conducteur.

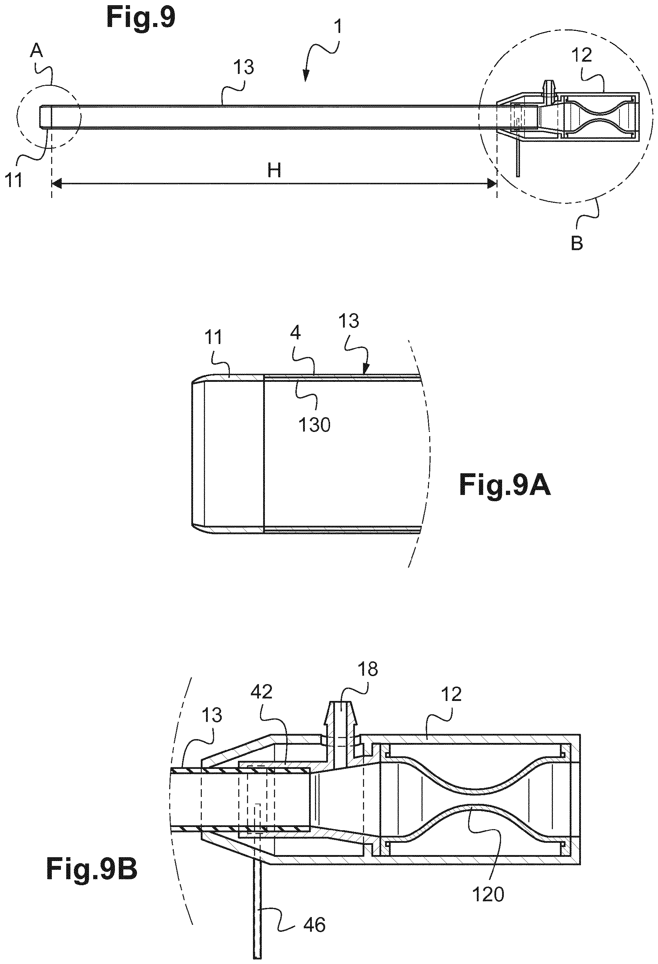

Les figures 9 à 9B illustrent un autre mode de réalisation avantageux d'un introducteur 1 selon l'invention qui a fait l'objet d'un prototype par l'inventeur.

Cet introducteur 1 comprend un revêtement bi-couche avec une couche interne 130 isolante électrique et un revêtement conducteur électrique 4 déposé sur la couche interne 130. Ce revêtement conducteur 4 forme l'élément conducteur destiné à être en contact avec le tissu sous-cutané du corps d'un patient. Il peut s'agir avantageusement d'un revêtement en carbone conducteur.

De préférence, comme illustré en figure 9B, le revêtement conducteur électrique est déposé sur toute la hauteur H de la périphérie extérieure 130 de la gaine (13) à l'exception de l'extrémité distale 11 de cette dernière.

L'extrémité proximale de l'introducteur 1 comprend quant à elle un embout 12 intégrant en son sein une bague 42 dans laquelle la gaine 13 est emmanchée à force.

L'embout 12 comprend notamment une valve 120 et un embout 18 pour la purge fluidique au moyen d'un liquide de rinçage approprié.

L'embout 12 comprend également une languette de connexion 46 en matériau conducteur électrique, dont une extrémité est insérée à travers la bague 42 de sorte à établir un contact électrique avec le revêtement électrique conducteur 4, et dont l'autre extrémité fait saillie à l'extérieur de l'embout.

Dans l'exemple illustré, l'extrémité de la languette 46 à l'extérieur de l'embout 12 s'étend transversalement à l'axe de l'introducteur 1 dans sa configuration non contrainte à l'intérieur du corps d'un patient. Cette languette de connexion 46 et ainsi agencée pour simplifier au mieux la mise en place et le maintien de la pince de connexion 5 qui peuvent se faire du côté du stimulateur cardiaque extérieur. Il est donc plus aisé pour médecin interventionniste de contrôler à la fois le maintien de la pince de connexion et le stimulateur cardiaque extérieur.

La surface de l'extrémité de la languette de connexion 46 à l'extérieur de l'embout 12 est adaptée pour être serrée par une pince de connexion 5 destinée à être reliée à une électrode d'un stimulateur cardiaque.

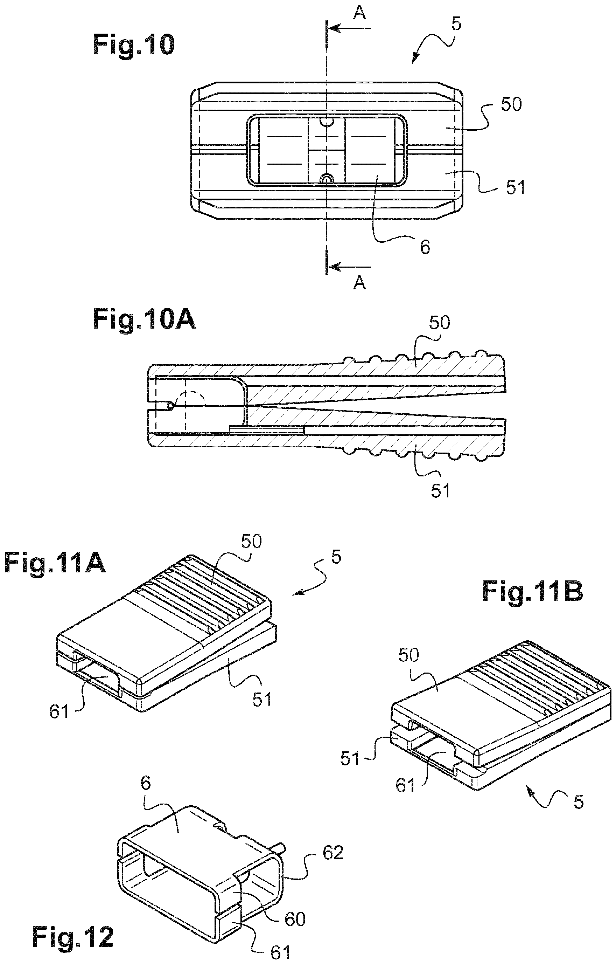

On a représenté en figures 10 et 10A, une pince de connexion 5 particulièrement avantageuse, car ergonomique, compacte et qui peut se serrer facilement et de manière fiable sur la languette de connexion 46.

Cette pince 5 comprend deux branches 50, 51 isolantes électriquement articulées l'une à l'autre et qui loge une pièce de clip 6 en matériau conducteur électrique.

Comme monté en figure 12, ce clip 6 comprend également deux branches 60, 61 formant des mâchoires, réunies entre elles au moyen d'un fond 62 en U. La flexibilité de ce clip 6 permet d'écarter les branches 60, 61 l'une de l'autre, l'absence d'effort sur le clip 6 mettant les branches 60, 61 en contact l'une de l'autre.

Ainsi, grâce à un simple appui manuel sur les branches 50, 51 de la pince 5 permet de faire passer celle-ci de sa position de fermeture (figure 11A) dans laquelle les mâchoires 60, 61 peuvent venir se serrer autour de la languette de connexion 46 de l'introducteur afin d'établir une connexion électrique avec le stimulateur cardiaque externe, à sa position d'ouverture (figure 11B) qui permet de libérer la pince 5 de la languette de connexion 46.

### Références citées

[1]: « Registry of Transcatheter Aortic-Valve Implantation in High-Risk Patients », Gilard et al; the New England Journal of Medicine : pp 1705-1715
[2]: « Left Ventricular Guidewire Pacing to Simplify Aortic Balloon Valvuloplasty », Susanne Navarini et al; Catheterization and Cardiovascular Interventions 73: pp 426-427 (2009)
[3]: « A novel Approach for Transcoronary Pacing un a Porcine Model », Roland Prodzinsky et al ; Journal of Invasive Cardiology 24(9) : pp 451-455 (2012)
[4]: « Optimizing of Transcoronary Pacing in a Porcine Model », Konstantin M. Heinroth, et al, Journal of Invasive Cardiology 21, pp 634-638 (2009)

## Revendications

1. Ensemble de remplacement d'une valve cardiaque par voie percutanée, comprenant :
- un dispositif formant un introducteur (1) comprenant au moins une gaine tubulaire (13) d'introduction, destinée à être introduite dans une artère d'un corps humain (C) et à laisser passer un dispositif d'intervention chirurgicale, tel qu'un cathéter de délivrance, et au moins un élément conducteur électrique (4) dont une portion distale (4a) est apparente sur une au moins une partie de la périphérie extérieure de la gaine de sorte à être en contact avec le tissu sous-cutané du corps ou avec l'artère et dont une portion proximale (4b) est accessible depuis l'extérieur du corps (C) de sorte à servir de connexion à une électrode d'un stimulateur cardiaque ;
- au moins un guide-fil (20) destiné à être introduit dans la gaine tubulaire (13) de l'introducteur pour l'avancement d'une valve artificielle destinée à remplacer la valve cardiaque, le guide-fil (20) comprenant une partie métallique servant en outre de connexion à l'autre électrode du stimulateur cardiaque.

2. Ensemble de remplacement d'une valve cardiaque par voie percutanée, comprenant :
- un dispositif formant un cathéter de délivrance de valve (1') comprenant au moins une gaine tubulaire (13) d'introduction, destinée à être introduit dans une artère d'un corps humain, et au moins un élément conducteur électrique (4) dont une portion distale (4a) est apparente sur une au moins une partie de la périphérie extérieure de la gaine de sorte à être en contact avec le tissu sous-cutané du corps ou avec l'artère et dont une portion proximale (4b) est accessible depuis l'extérieur du corps (C) de sorte à servir de connexion à une électrode d'un stimulateur cardiaque ;
- au moins un guide-fil (20) destiné à être introduit dans la gaine tubulaire (13) du cathéter de délivrance pour l'avancement d'une valve artificielle destinée à remplacer la valve cardiaque, le guide-fil comprenant au moins une partie métallique (20) servant en outre de connexion à l'autre électrode du stimulateur cardiaque.

3. Ensemble selon la revendication 1 ou 2, l'électrode du stimulateur cardiaque connectée à l'élément conducteur électrique (4) de l'introducteur (1) ou du cathéter de délivrance étant l'anode tandis que celle connectée à la partie métallique du guide-fil (20) est la cathode.

4. Ensemble selon l'une des revendications 1 à 3, l'élément conducteur électrique étant un fil ou une bande métallique (4) logé(e) au moins en partie dans l'épaisseur de la gaine dont une portion distale (4a) est apparente à la périphérie extérieure de la gaine.

5. Ensemble selon la revendication 4, l'introducteur ou le cathéter de délivrance comprenant :
- une échancrure distale (17) réalisée dans l'épaisseur de la gaine tubulaire (13) et laissant apparente la portion distale (4a) de fil ou de bande métallique de sorte à réaliser le contact avec une paroi du système vasculaire du corps ;
- une échancrure proximale, réalisée dans une zone proximale (Z_{E}) de l'introducteur destinée à être à l'extérieur du corps, et laissant apparente la portion proximale (4b) de fil ou de bande métallique de sorte à réaliser la connexion avec l'électrode du stimulateur cardiaque.

6. Ensemble selon la revendication 4 ou 5, comprenant un dispositif de rinçage (16) à robinets, dit « flush », pour rincer l'intérieur de l'introducteur (1) ou du cathéter de délivrance (1') au moyen d'un liquide de rinçage approprié, la portion proximale (4b) du fil ou de la bande métallique (4) étant apparente au niveau du flush (16).

7. Ensemble selon la revendication 5 ou 6, l'échancrure proximale et la portion proximale (4b) de fil ou bande métallique étant conformées pour permettre le pincement d'une pince de type crocodile sur ladite portion.

8. Ensemble selon l'une des revendications 4 à 7, la portion distale (4a) du fil ou bande métallique est agencée :
- soit à une distance D, mesurée depuis l'extrémité proximale de l'introducteur, comprise entre la moitié et les trois quart de la hauteur de la portion tubulaire (15) de la gaine définissant la zone proximale (Z_{E}) de l'introducteur ;
- soit à une distance comprise entre 20 et 60cm de la zone distale du cathéter de délivrance.

9. Ensemble selon l'une des revendications 4 à 8, la section du fil ou de la bande métallique étant comprise entre 0,25 et 5 mm².

10. Ensemble selon l'une des revendications 1 à 3, l'élément conducteur électrique comprenant un revêtement conducteur électrique (4), tel qu'un revêtement en carbone, déposé sur la périphérie extérieure de la gaine (13).

11. Ensemble selon la revendication 10, la gaine (13) étant un revêtement bi-couche avec une couche interne (130) isolante électrique et le revêtement conducteur électrique (4) déposé sur la couche interne (130).

12. Ensemble selon la revendication 10 ou 11, le revêtement conducteur électrique étant déposé sur toute la hauteur (H) de la périphérie extérieure (130) de la gaine (13) à l'exception de l'extrémité distale (11) de cette dernière.

13. Ensemble selon l'une des revendications 10 à 12, comprenant :
- un embout (12) intégrant en son sein une bague (42) dans laquelle la gaine est emmanchée à force,
- une languette de connexion (46) en matériau conducteur électrique, dont une extrémité est insérée à travers la bague de sorte à établir un contact électrique avec le revêtement électrique conducteur (4), et dont l'autre extrémité fait saillie à l'extérieur de l'embout, la surface de cette extrémité à l'extérieur de l'embout étant adaptée pour être serrée par une pince de connexion (5) destinée à être reliée à une électrode d'un stimulateur cardiaque.

## Patentansprüche

1. Anordnung zum Ersetzen einer Herzklappe auf perkutanem Wege, umfassend:
- eine einen Einführer (1) bildende Vorrichtung, die mindestens eine röhrenförmige Einführungshülse (13), die dazu bestimmt ist, in eine Arterie eines menschlichen Körpers (C) eingeführt zu werden und eine chirurgische Eingriffsvorrichtung wie einen Zuführkatheter hindurchgehen zu lassen, und mindestens ein Elektrizitätsleiterelement (4) umfasst, von dem ein distaler Abschnitt (4a) auf mindestens einem Teil des Außenumfangs der Hülse freiliegt, so dass er mit dem Unterhautgewebe des Körpers oder mit der Arterie in Kontakt ist, und von dem ein proximaler Abschnitt (4b) von außerhalb des Körpers (C) zugänglich ist, um als Anschluss an eine Elektrode eines Herzschrittmachers zu dienen,
- mindestens einen Führungsdraht (20), der dazu bestimmt ist, zum Vorschieben einer zum Ersatz der Herzklappe bestimmten künstlichen Klappe in die röhrenförmige Hülse (13) des Einführers eingeführt zu werden, wobei der Führungsdraht (20) einen metallischen Teil umfasst, der ferner als Anschluss an die andere Elektrode des Herzschrittmachers dient.

2. Anordnung zum Ersetzen einer Herzklappe auf perkutanem Wege, umfassend:
- eine einen Klappenzuführkatheter (1') bildende Vorrichtung, die mindestens eine röhrenförmige Einführungshülse (13), die dazu bestimmt ist, in eine Arterie eines menschlichen Körpers eingeführt zu werden, und mindestens ein Elektrizitätsleiterelement (4) umfasst, von dem ein distaler Abschnitt (4a) auf mindestens einem Teil des Außenumfangs der Hülse freiliegt, so dass er mit dem Unterhautgewebe des Körpers oder mit der Arterie in Kontakt ist, und von dem ein proximaler Abschnitt (4b) von außerhalb des Körpers (C) zugänglich ist, um als Anschluss an eine Elektrode eines Herzschrittmachers zu dienen,
- mindestens einen Führungsdraht (20), der dazu bestimmt ist, zum Vorschieben einer zum Ersatz der Herzklappe bestimmten künstlichen Klappe in die röhrenförmige Hülse (13) des Zuführkatheters eingeführt zu werden, wobei der Führungsdraht mindestens einen metallischen Teil (20) umfasst, der ferner als Anschluss an die andere Elektrode des Herzschrittmachers dient.

3. Anordnung nach Anspruch 1 oder 2, wobei die mit dem Elektrizitätsleiterelement (4) des Einführers (1) oder des Zuführkatheters verbundene Elektrode des Herzschrittmachers die Anode ist, während die mit dem metallischen Teil des Führungsdrahts (20) verbundene die Kathode ist.

4. Anordnung nach einem der Ansprüche 1 bis 3, wobei das Elektrizitätsleiterelement ein Metalldraht oder ein Metallsteg (4) ist, der mindestens teilweise in der Dicke der Hülse aufgenommen ist und von dem ein distaler Abschnitt (4a) auf dem Außenumfang der Hülse freiliegt.

5. Anordnung nach Anspruch 4, wobei der Einführer oder der Zuführkatheter Folgendes umfasst:
- eine distale Kerbe (17), die in der Dicke der röhrenförmige Hülse (13) ausgeführt ist und den distalen Abschnitt (4a) des Metalldrahts oder -stegs frei lässt, um den Kontakt mit einer Wand des Gefäßsystems des Körpers herzustellen,
- eine proximale Kerbe, die in einem proximalen Bereich (Z_{E}) des Einführers, der dazu bestimmt ist, außerhalb des Körpers zu liegen, ausgeführt ist und den proximalen Abschnitt (4b) des Metalldrahts oder -stegs frei lässt, um den Kontakt mit der Elektrode des Herzschrittmachers herzustellen.

6. Anordnung nach Anspruch 4 oder 5, umfassend eine Spülvorrichtung (16) mit Hähnen, die als Flush bezeichnet wird, zum Spülen des Inneren des Einführers (1) oder des Zuführkatheters (1') mittels einer geeigneten Spülflüssigkeit, wobei der proximale Abschnitt (4b) des Metalldrahts oder -stegs (4) am Flush (16) freiliegt.

7. Anordnung nach Anspruch 5 oder 6, wobei die proximale Kerbe und der proximale Abschnitt (4b) des Metalldrahts oder -stegs so ausgestaltet sind, dass eine Krokodilklammer an diesen Abschnitt geklammert werden kann.

8. Anordnung nach einem der Ansprüche 4 bis 7, wobei der distale Abschnitt (4a) des Metalldrahts oder -stegs wie folgt angeordnet ist:
- entweder mit einem vom proximalen Ende des Einführers gemessenen Abstand D zwischen der Hälfte und drei Viertel der Höhe des röhrenförmigen Abschnitts (15) der Hülse, der den proximalen Bereich (Z_{E}) des Einführers definiert,
- oder mit einem Abstand zwischen 20 und 60 cm von dem distalen Bereich des Zuführkatheters.

9. Anordnung nach einem der Ansprüche 4 bis 8, wobei der Querschnitt des Metalldrahts oder -stegs zwischen 0,25 und 5 mm² beträgt.

10. Anordnung nach einem der Ansprüche 1 bis 3, wobei das Elektrizitätsleiterelement eine elektrisch leitende Beschichtung (4) wie eine Kohlenstoffbeschichtung umfasst, die auf dem Außenumfang der Hülse (13) aufgebracht ist.

11. Anordnung nach Anspruch 10, wobei die Hülse (13) eine zweilagige Beschichtung ist, mit einer elektrisch isolierenden inneren Lage (130) und der auf der inneren Lage (130) aufgebrachten elektrisch leitenden Beschichtung (4).

12. Anordnung nach Anspruch 10 oder 11, wobei die elektrisch leitende Beschichtung auf die gesamte Höhe (H) des Außenumfangs (130) der Hülse (13) mit Ausnahme des distalen Endes (11) der Letzteren aufgebracht ist.

13. Anordnung nach einem der Ansprüche 10 bis 12, umfassend:
- ein Anschlussstück (12), in dem ein Ring (42) integriert ist, in dem die Hülse eingepresst ist,
- eine Verbindungszunge (46) aus elektrisch leitendem Material, deren eines Ende durch den Ring eingeführt ist, um einen elektrischen Kontakt mit der elektrisch leitenden Beschichtung (4) herzustellen, und deren anderes Ende außerhalb des Anschlussstücks vorragt, wobei die Oberfläche dieses Endes außerhalb des Anschlussstücks dazu ausgeführt ist, mit einer Verbindungsklemme (5) geklemmt zu werden, die zum Anschluss an eine Elektrode eines Herzschrittmachers bestimmt ist.

## Claims

1. An assembly for replacing a heart valve by a percutaneous route, comprising:
- a device forming an introducer (1) comprising at least one tubular insertion sheath (13), intended to be introduced into an artery of a human body (C) and to allow the passage of a surgical intervention device such as a delivery catheter, and at least one electrically conductive element (4), of which a distal portion (4a) is exposed on at least one part of the outer periphery of the sheath in such a way as to be in contact with the subcutaneous tissue of the body or with the artery, and of which a proximal portion (4b) is accessible from the outside of the body (C) in such a way as to serve as connection to one electrode of a cardiac stimulator;
- at least one guide wire (20) intended to be introduced into the tubular sheath (13) of the introducer for advancing an artificial valve intended to replace the heart valve, the guide wire (20) comprising a metallic part serving additionally as connection to the other electrode of the cardiac stimulator.

2. An assembly for replacing a heart valve by a percutaneous route, comprising:
- a device forming a valve delivery catheter (1') comprising at least one tubular insertion sheath (13), intended to be introduced into an artery of a human body, and at least one electrically conductive element (4), of which a distal portion (4a) is exposed on at least one part of the outer periphery of the sheath in such a way as to be in contact with the subcutaneous tissue of the body or with the artery, and of which a proximal portion (4b) is accessible from the outside of the body (C) in such a way as to serve as connection to one electrode of a cardiac stimulator;
- at least one guide wire (20) intended to be introduced into the tubular sheath (13) of the delivery catheter for advancing an artificial valve intended to replace the heart valve, the guide wire comprising at least one metallic part (20) serving additionally as connection to the other electrode of the cardiac stimulator.

3. The assembly as claimed in claim 1 or 2, the electrode of the cardiac stimulator connected to the electrically conductive element (4) of the introducer (1) or of the delivery catheter being the anode, while the electrode connected to the metallic part of the guide wire (20) is the cathode.

4. The assembly as claimed in one of claims 1 through 3, the electrically conductive element being a metal wire or strip (4) which is accommodated at least in part within the thickness of the sheath, and of which a distal portion (4a) is exposed at the outer periphery of the sheath.

5. The assembly as claimed in claim 4, the introducer or the delivery catheter comprising:
- a distal notch (17) formed within the thickness of the tubular sheath (13) and leaving the distal portion (4a) of the metal wire or strip exposed, so as to produce the contact with a wall of the vascular system of the body;
- a proximal notch formed in a proximal zone (Z_{E}) of the introducer, intended to be outside the body, and leaving the proximal portion (4b) of the metal wire or strip exposed, so as to produce the connection to the electrode of the cardiac stimulator.

6. The assembly as claimed in claim 4 or 5, comprising a rinsing device (16) with faucets, called a flush, for rinsing the inside of the introducer (1) or of the delivery catheter (1') by means of a suitable rinsing liquid, the proximal portion (4b) of the metal wire or strip (4) being exposed at the level of the flush (16).

7. The assembly as claimed in claim 5 or 6, the proximal notch and the proximal portion (4b) of the metal wire or strip being configured to allow a crocodile clip to be clipped onto said portion.

8. The assembly as claimed in one of claims 4 through 7, the distal portion (4a) of the metal wire or strip being arranged:
- either at a distance D, measured from the proximal end of the introducer, of between half and three quarters the height of the tubular portion (15) of the sheath defining the proximal zone (Z_{E}) of the introducer;
- or at a distance of between 20 and 60 cm from the distal zone of the delivery catheter.

9. The assembly as claimed in one of claims 4 through 8, the cross section of the metal wire or strip being between 0.25 and 5 mm².

10. The assembly as claimed in one of claims 1 through 3, the electrically conductive element comprising an electrically conductive covering (4), such as a carbon covering, deposited on the outer periphery of the sheath (13).

11. The assembly as claimed in claim 10, the sheath (13) being a two-layer covering with an electrically insulating inner layer (130) and the electrically conductive layer (4) deposited on the inner layer (130).

12. The assembly as claimed in claim 10 or 11, the electrically conductive covering being deposited over the entire height (H) of the outer periphery (130) of the sheath (13), except for the distal end (11) of the latter.

13. The assembly as claimed in one of claims 10 through 12, comprising:
- a nozzle (12) and, integrated within the latter, a ring (42) in which the sheath is engaged with force,
- a connecting tongue (46) made of electrically conductive material, of which one end is inserted through the ring in such a way as to establish an electrical contact with the electrically conductive covering (4), and of which the other end protrudes outside the nozzle, the surface of this end outside the nozzle being designed to be clamped by a connecting clip (5) intended to be joined to an electrode of a cardiac stimulator.
